# EUROPÄISCHE PATENTSCHRIFT

(11) **EP 1 415 649 B1**
(45) Veröffentlichungstag und Bekanntmachung des Hinweises auf die Patenterteilung: **04.03.2009**
(21) Anmeldenummer: 03023846.3
(22) Anmeldetag: 20.10.2003
(51) Int. Cl.: A61K 9/70, A61K 31/216, A61K 47/46

(54) **Transdermales Wirkstoffabgabesystem für Oxybutynin**
Transdermal therapeutic system comprising oxybutynin
Système thérapeutique transdermique d'oxybutynin

(30) Priorität: 04.11.2002 DE 10251256
(43) Veröffentlichungstag der Anmeldung: 06.05.2004
(73) Patentinhaber: Acino AG, 83714 Miesbach (DE)
(72) Erfinder: Finckh, Matthias, Dr., 81547 München (DE); Tisa-Bostedt, Katalin, Dr., 82049 Pullach (DE); Fischbacher, Eva, 83075 Bad Feilnbach (DE)
(74) Vertreter: Boeters, Hans Dietrich

(56) Entgegenhaltungen:
- WO-A-01/68138
- WO-A-95/09007
- US-A- 5 601 839

## Beschreibung

WO 99/48 493 beschreibt ein Oxybutyninpflaster, das gemäß dem sogenannten Heißschmelzverfahren hergestellt wurde. Es ist angeben, daß das Pflaster keine Resorptionsverstärker enthält. Trotzdem werden Substanzen insbesondere Zitronensäuretriester erwähnt, welche normalerweise als Resorptionsverstärker verwendet werden.

US 5 601 839 beschreibt Triacetin als ein permeationsförderndes Mittel.

WO 01/68138 beschreibt eine topische Formulierung, beispielsweise ein Pflaster, das eine Klebeschicht aus beispielsweise Acrylat, einen Wirkstoff und eine Aloe-Zusammensetzung zur Förderung der intradermalen Absorption enthält.

Hinsichtlich der Oxybutynin-Pflaster sollten auch US 5 411 740 und WO 93/23 025 erwähnt werden.

Das die Erfindung betreffende Problem wird gelöst durch ein Transdermales Wirkstoffabgabesystem (TDS) umfassend:
- eine für den Wirkstoff undurchlässige Abdeckung,
- eine Matrix, die Oxybutynin als Wirkstoff enthält, und
- eine fakultative Abziehfolie (Release Liner),
wobei die Matrix weiterhin
- einen Aloe Vera-Extrakt
- einen druckempfindlichen Klebstoff und
- einen Vernetzer für den Klebstoff
umfaßt.

Das erfindungsgemäße transdermale Wirkstoffabgabesystem kann racemisches Oxybutynin, R-Oxybutynin, S-Oxybutynin oder Desethyl-Oxybutynin umfassen.

Ferner kann der druckempfindliche Klebstoff des erfindungsgemäßen transdermalen Wirkstoffabgabesystems umfassen oder bestehen aus einem auf Acrylat basierenden Polymer, vorzugsweise einen/m Polymer, das auf einem Acrylat-Vinylacetat-Copolymer basiert.

Ferner kann der druckempfindliche Klebstoff des erfindungsgemäßen transdermalen Wirkstoffabgabesystems umfassen oder bestehen aus Durotak 2287 oder Durotak 2516.

Ferner kann die Matrix des erfindungsgemäßen transdermalen Wirkstoffabgabesystems Ti-Acetylacetonat, Al-Acetylacetonat oder Polybutyltitanat als Vernetzer umfassen.

Ferner kann das Extraktionsmittel des Aloe Vera-Extrakts des erfindungsgemäßen transdermalen Wirkstoffabgabesystems ein Pflanzenöl, vorzugsweise ein Sojaöl sein.

Ein Aloe Vera-Extrakt ist zum Beispiel von Caesar & Loretz (Hilden/Deutschland) erhältlich.

Ferner kann der Aloe Vera-Extrakt des erfindungsgemäßen transdermalen Wirkstoffabgabesystems 5 bis 15 Gew.-% Aloe Vera-Öl und 95 bis 85 Gew.-% Pflanzenöl umfassen.

Ferner kann die Matrix des erfindungsgemäßen transdermalen Wirkstoffabgabesystems den Aloe Vera-Extrakt als einzigen Resorptionsverstärker enthalten.

Ferner kann die Matrix des erfindungsgemäßen transdermalen Wirkstoffabgabesystems 5 bis 40 Gew.-%, vorzugsweise 10 bis 35 Gew.-% und insbesondere 15 bis 30 Gew.-% Oxybutynin (basierend auf der Matrix) enthalten.

Ferner kann die Matrix des erfindungsgemäßen transdermalen Wirkstoffabgabesystems 10 bis 25 Gew.-%, vorzugsweise 12 bis 20 und insbesondere 14 bis 18 Gew.-% des Aloe Vera-Extrakts (basierend auf der Matrix) umfassen.

Ferner kann die Matrix des erfindungsgemäßen transdermalen Wirkstoffabgabesystems 0,1 bis 5,0 Gew.-%, vorzugsweise 0,3 bis 3 und insbesondere 0,5 bis 2,0 Gew.-% des Vernetzers (basierend auf der Matrix) enthalten.

Das erfindungsgemäße transdermale Wirkstoffabgabesystem kann eine Oberfläche von 5 bis 80 cm², vorzugsweise von 10 bis 60 cm² und insbesondere von 20 bis 50 cm² haben.

### Beispiele und Vergleichsbeispiel:

Eine Zusammensetzung einer erfindungsgemäßen Matrix wurde wie folgt zur Verfügung gestellt:

| | |
|---|---|
| Oxybutynin: | 20,0 % |
| Aloe Vera-Extrakt (Sojabohnenöl) | 15,0 % |
| Ti-Acetylacetonat (Tyzor AA 75) | 1,3 % |
| Durotak 2287 | Rest |

Diese Zusammensetzung wurde einem Permeationstest unterworfen (Mäusehaut). Der maximale Flux betrug 9,2 µg/cm² · h. Die Permeation betrug 190 µg/cm²·24 h.

Gemäß US 5 601 839 wurde eine Matrix mit der folgenden Zusammensetzung zur Verfügung gestellt:

| | |
|---|---|
| Oxybutynin | 20,0 % |
| Triacetin | 15,0 % |
| Al-Acetylacetonat | 0,5 % |
| Durotak 2051 (Acrylat/Vinylacetat-Klebstoff) | Rest |

Diese Zusammensetzung wurde auch einem Permeationstest (Mäusehaut) unterworfen. Der maximale Flux betrug 5,3 µg/cm²·h. Die Permeation betrug 80 µg/cm² 24 h.

## Patentansprüche

1. Transdermales Wirkstoffabgabesystem (TDS) umfassend:
- eine für den Wirkstoff undurchläßige Abdeckung
- eine Matrix, die Oxybutynin als Wirkstoff enthält, und
- eine fakultative Abziehfolie (Release-Liner),
- wobei die Matrix weiterhin,
- einen Aloe Vera-Extrakt,
- einen druckempfindlichen Klebstoff und
- einen Vernetzer für den Klebstoff
umfaßt.

2. Transdermales Wirkstoffabgabesystem gemäß Anspruch 1, umfassend racemisches Oxybutynin, R-Oxybutynin, S-Oxybutynin oder Desethyl-Oxybutynin.

3. Transdermales Wirkstoffabgabesystem gemäß Anspruch 1 oder 2, wobei der druckempfindliche Klebstoff der Matrix umfaßt oder besteht aus einem auf Acrylat basierenden Polymer, vorzugsweise einem Polymer, das auf einem Acrylat-Vinylacetat-Copolymer basiert.

4. Transdermales Wirkstoffabgabesystem gemäß irgendeinem der vorstehenden Ansprüche, wobei die Matrix umfaßt oder besteht aus Durotak 2287 oder Durotak 2516.

5. Transdermales Wirkstoffabgabesystem gemäß irgendeinem der vorstehenden Ansprüche, wobei die Matrix Ti-Acetylacetonat, Al-Acetylacetonat oder Polybutyl-Titanat als Vernetzer umfaßt.

6. Transdermales Wirkstoffabgabesystem gemäß irgendeinem der vorstehenden Ansprüche, wobei das Extraktionsmittel des Aloe Vera-Extrakts ein Pflanzenöl, vorzugsweise Sojaöl ist.

7. Transdermales Wirkstoffabgabesystem gemäß Anspruch 6, wobei der Aloe Vera-Extrakt 5 bis 15 Gew.-% des Aloe Vera-Öls und 95 bis 85 Gew.-% des Pflanzenöls umfaßt.

8. Transdermales Wirkstoffabgabesystem gemäß irgendeinem der vorstehenden Ansprüche, wobei die Matrix den Aloe Vera-Extrakt als einzigen Resorptionsverstärker umfaßt.

9. Transdermales Wirkstoffabgabesystem gemäß irgendeinem der vorstehenden Ansprüche, wobei die Matrix 5 bis 40 Gew.-%, vorzugsweise 10 bis 35 Gew.-% und insbesondere 15 bis 30 Gew.-% Oxybutynin (bezogen auf die Matrix) umfaßt.

10. Transdermales Wirkstoffabgabesystem gemäß irgendeinem der vorstehenden Ansprüche, wobei die Matrix 10 bis 25 Gew.-%, vorzugsweise 12 bis 20 Gew.-% und insbesondere 14 bis 18 Gew.-% Aleo Vera-Extrakt (auf die Matrix bezogen) umfaßt.

11. Transdermales Wirkstoffabgabesystem gemäß irgendeinem der vorstehenden Ansprüche, wobei die Matrix 0,1 bis 5,0 Gew.-%, vorzugsweise 0,3 bis 3 Gew.-% und insbesondere 0,5 bis 2,0 Gew.-% des Vernetzers (auf die Matrix bezogen) umfaßt.

12. Transdermales Wirkstoffabgabesystem gemäß irgendeinem der vorstehenden Ansprüche, wobei das System eine Oberfläche von 5 bis 80, vorzugsweise 10 bis 60 und insbesondere 20 bis 50 cm² besitzt.

## Claims

1. Transdermal drug delivery system (TDS) comprising
- a cover which is impermeable for the active ingredient,
- a matrix containing oxybutynin as active ingredient and
- facultative release liner,
wherein the matrix further comprises
- an Aloe Vera extract,
- a pressure sensitive adhesive and
- a cross linking agent for the adhesive.

2. Transdermal drug delivery system according to claim 1, comprising racemic oxybutynin, R-oxybutynin, S-oxybutynin or desethyl-oxybutynin.

3. Transdermal drug delivery system according to claim 1 or 2, wherein the pressure sensitive adhesive of the matrix comprises or consists of an acrylate based polymer, preferably a polymer based on an acrylate-vinyl acetate copolymer.

4. Transdermal drug delivery system according to any of the preceding claims, wherein the matrix comprises or consists of Durotak 2287 or Durotak 2516.

5. Transdermal drug delivery system according to any of the preceding claims, wherein the matrix comprises Ti-acetylacetonate, A1-acetylacetonate or polybutyl-titanate as crosslinking agent.

6. Transdermal drug delivery system according to any of the preceding claims, wherein the extracting agent of the Aloe Vera-extract is a vegetable oil, preferably soybean oil.

7. Transdermal drug delivery system according to claim 6, wherein the Aloe Vera-extract comprises 5 to 15 % by weight of Aloe Vera oil and 95 to 85 % by weight of vegetable oil.

8. Transdermal drug delivery system according to any of the preceding claims, wherein the matrix comprises the Aloe Vera-extract as the only resorption-enhancer.

9. Transdermal drug delivery system according to any of the preceding claims, wherein the matrix comprises 5 to 40, preferably 10 to 35 and especially 15 to 30 % by weight of oxybutynin (based on the matrix).

10. Transdermal drug delivery system according to any of the preceding claims, wherein the matrix comprises 10 to 25, preferably 12 to 20 and especially 14 to 18 % by weight of Aloe Vera-extract (based on the matrix).

11. Transdermal drug delivery system according to any of the preceding claims, wherein the matrix comprises 0.1 to 5.0, preferably 0.3 to 3 and especially 0.5 to 2.0 % by weight of the crosslinking agent (based on the matrix).

12. Transdermal drug delivery system according to any of the preceding claims, wherein the system has a surface of 5 to 80, preferably 10 to 60 and especially 20 to 50 cm².

## Revendications

1. Système transdermique de distribution de principes actifs (TDS), comprenant :
- un recouvrement impénétrable pour le principe actif,
- une matrice contenant de l'oxybutynine à titre de principe actif, et
- un film détachable (release liner) facultatif,
- où la matrice contient en outre
- un extrait d'Aloe Vera,
- un adhésif sensible à la pression et
- un réticulant pour l'adhésif.

2. Système transdermique de distribution de principes actifs selon la revendication 1, comprenant de l'oxybutynine racémique, de la R-oxybutynine, de la S-oxybutynine ou de la déséthyloxybutynine.

3. Système transdermique de distribution de principes actifs selon la revendication 1 ou 2, dans lequel l'adhésif sensible à la pression de la matrice comprend ou se compose d'un polymère à base d'acrylate, de préférence un polymère qui est à base d'un copolymère d'acrylate - acétate de vinyle.

4. Système transdermique de distribution de principes actifs selon l'une quelconque des revendications précédentes, dans lequel la matrice comprend ou se compose de Durotak 2287 ou de Durotak 2516.

5. Système transdermique de distribution de principes actifs selon l'une quelconque des revendications précédentes, dans lequel la matrice comprend de l'acétylacétonate de Ti, de l'acétylacétonate d'Al ou du titanate de polybutyle à titre de réticulant.

6. Système transdermique de distribution de principes actifs selon l'une quelconque des revendications précédentes, dans lequel l'agent d'extraction de l'extrait d'Aloe Vera est une huile végétale, de préférence l'huile de soja.

7. Système transdermique de distribution de principes actifs selon la revendication 6, dans lequel l'extrait d'Aloe Vera comprend 5 à 15 % en poids d'huile d'Aloe Vera et 85 à 95 % en poids d'huile végétale.

8. Système transdermique de distribution de principes actifs selon l'une quelconque des revendications précédentes, dans lequel la matrice comprend l'extrait d'Aloe Vera à titre d'unique renforçateur de résorption.

9. Système transdermique de distribution de principes actifs selon l'une quelconque des revendications précédentes, dans lequel la matrice comprend 5 à 40 % en poids, de préférence 10 à 35 % en poids et en particulier 15 à 30 % en poids d'oxybutynine (par rapport à la matrice).

10. Système transdermique de distribution de principes actifs selon l'une quelconque des revendications précédentes, dans lequel la matrice comprend 10 à 25 % en poids, de préférence 12 à 20 % en poids et en particulier 14 à 18 % en poids d'extrait d'Aloe Vera (par rapport à la matrice).

11. Système transdermique de distribution de principes actifs selon l'une quelconque des revendications précédentes, dans lequel la matrice comprend 0,1 à 5,0 % en poids, de préférence 0,3 à 3 % en poids et en particulier 0,5 à 2,0 % en poids de réticulant (par rapport à la matrice).

12. Système transdermique de distribution de principes actifs selon l'une quelconque des revendications précédentes, dans lequel le système possède une surface de 5 à 80, de préférence de 10 à 60 et en particulier de 20 à 50 cm².
